# EUROPÄISCHE PATENTANMELDUNG

(11) **EP 4 628 138 A1**
(43) Veröffentlichungstag der Anmeldung: **08.10.2025**
(21) Anmeldenummer: 25167954.4
(22) Anmeldetag: 02.04.2025
(51) Int. Cl.: A61M 25/00, A61M 25/01

(54) **KATHETERANORDNUNG FÜR EINE REGIONALANÄSTHESIE**

(30) Priorität: 03.04.2024 DE 102024109245
(71) Anmelder: B. Braun Melsungen AG, 34212 Melsungen (DE)
(72) Erfinder: Henschel, Cristiane, 34117 Kassel (DE); Berg, Jacqueline, 34212 Melsungen (DE); Bolz, Johannes, 34132 Kassel (DE); Claus, Benjamin, 34131 Kassel (DE); Freudenstein, Manuel, 34253 Lohfelden (DE); Hohmann, Christian, 34295 Edermünde (DE); Klotzbach, Roman, 43225 Baunatal (DE)
(74) Vertreter: Patentanwälte Ruff, Wilhelm, Beier, Dauster & Partner mbB

(57) **Zusammenfassung**

Eine derartige Katheteranordnung (1) mit einem Kapillargehäuse (2), an dem distal ein Kapillarrohr (3) vorgesehen ist, sowie mit einem Katheterrohr (4), das proximal in das Kapillarrohr (3) eingeführt ist und distal aus dem Kapillarrohr (3) herausragt, wobei das Katheterrohr (4) in dem Kapillarrohr (3) begrenzt linearbeweglich verschiebbar angeordnet ist, sowie mit einer manuell betätigbaren Klemmeinrichtung zur lösbaren Fixierung des Katheterrohrs (4) relativ zu dem Kapillarrohr (3) und dem Kapillargehäuse (2) ist bekannt.

Erfindungsgemäß ist zusätzlich eine Verstelleinrichtung am Kapillargehäuse (2) vorgesehen, die eine stufenweise oder stufenlose Verschiebung des Katheterrohrs (4) im Kapillarrohr (3) gewährleistet, wobei an dem Katheterrohr (4) wenigstens ein manuell ergreifbarer radialer Überstand vorgesehen ist, der entlang des Kapillargehäuses (2) verstellbar geführt ist.

## Beschreibung

Die Erfindung betrifft eine Katheteranordnung für eine Regionalanästhesie, mit einem Kapillargehäuse, an dem distal ein Kapillarrohr vorgesehen ist, sowie mit einem Katheterrohr, das proximal in das Kapillarrohr eingeführt ist und distal abschnittsweise aus dem Kapillarrohr herausragt, wobei das Katheterrohr in dem Kapillarrohr begrenzt linearbeweglich verschiebbar angeordnet ist, sowie mit einer manuell betätigbaren Klemmeinrichtung zur lösbaren Fixierung des Katheterrohrs relativ zu dem Kapillarrohr und dem Kapillargehäuse.

Eine derartige Katheteranordnung ist aus der DE 10 2022 205 130 A1 bekannt. Die bekannte Katheteranordnung weist ein Kapillargehäuse und ein fest an dem Kapillargehäuse angebrachtes Kapillarrohr auf. Ein schlauchförmiges Katheterrohr ist in das Kapillarrohr eingeführt und ragt an einem distalen Rohrende des Kapillarrohrs axial über das Kapillarrohr hinaus. Zur Fixierung des Katheterrohrs relativ zum Kapillargehäuse ist eine Fixiervorrichtung vorgesehen, die ein Betätigungselement und ein Klemmelement aufweist. Das Klemmelement ist an dem Kapillargehäuse gelagert. Das Betätigungselement ist relativ zu dem Kapillargehäuse beweglich gelagert und kraft- und/oder bewegungsübertragend mit dem Klemmelement wirkverbunden, um eine axiale Klemmung des schlauchförmigen Katheterrohrs relativ zum Kapillarrohr oder eine Freigabe einer axialen Beweglichkeit des schlauchförmigen Katheterrohrs relativ zum Kapillarrohr zu ermöglichen.

Aufgabe der Erfindung ist es, eine Katheteranordnung der eingangs genannten Art zu schaffen, die einfach bedienbar ist.

Diese Aufgabe wird dadurch gelöst, dass zusätzlich eine Verstelleinrichtung am Kapillargehäuse vorgesehen ist, die eine stufenweise oder stufenlose Verschiebung des Katheterrohrs im Kapillarrohr gewährleistet, wobei an dem Katheterrohr wenigstens ein manuell ergreifbarer radialer Überstand, insbesondere ein Stellglied, vorgesehen ist, das entlang des Kapillargehäuses begrenzt verstellbar geführt ist. Die erfindungsgemäße Lösung ist für die Regionalanästhesie vorgesehen. Durch die am Kapillargehäuse vorgesehene Verstelleinrichtung kann ein Vorschub eines distal aus dem Kapillarrohr herausragenden Abschnitts des Katheterrohrs eingestellt werden und anschließend die eingestellte Vorschubposition mittels der Klemmeinrichtung fixiert werden. Sowohl die Verstelleinrichtung als auch die Klemmeinrichtung können durch einen Anwender mit einer Hand bedient werden, so dass der Anwender mit der anderen Hand frei ist, um eine zusätzliche Funktion auszuüben wie insbesondere das Halten und Führen eines Ultraschallgeräts. Durch die Veränderung des Vorschubs kann eine Spitze des Katheterrohrs besonders nahe an die zu anästhesierende Stelle, insbesondere an Nerven oder Nervengeflechte, herangeführt werden, um eine hohe Wirksamkeit der Regionalanästhesie zu erzielen. Das Katheterrohr kann zumindest abschnittsweise schlauchförmig ausgebildet sein. Zudem kann an das Katheterrohr ein Verlängerungsschlauch proximal angefügt sein. Wenn der Verlängerungsschlauch außen auf ein proximales Ende des Katheterrohrs aufgeschoben wird, bildet das distale Stirnende des Verlängerungsschlauches den radialen Überstand nach der Erfindung. Alternativ kann eine Perle oder ein ähnliches Stellglied auf dem Katheterrohr befestigt sein, um als radialer Überstand zu dienen. Der Begriff "radial" ist bezogen auf eine Längsachse des Katheterrohrs. Unter den Begriff des Katheterrohrs fällt auch ein solcher proximal an das in dem Kapillarrohr geführte Katheterrohr anschließender Verlängerungsschlauch, so dass der Überstand, insbesondere das Stellglied, auch an dem Verlängerungsschlauch vorgesehen sein kann. Das Stellglied umschließt vorzugsweise einen Bereich des Katheterrohrs insbesondere auf Höhe des Kapillargehäuses kraft-, form- oder stoffschlüssig, so dass eine Verstellung des Stellglieds zwangsläufig eine komplementäre Verlagerung des Katheterrohrs bewirkt. Bei einer stufenweisen Verschiebung des Katheterrohrs sind vorzugsweise an dem Kapillargehäuse wenigstens zwei Stufenprofilierungen vorgesehen, in die das Stellglied lösbar einrasten kann. Bei einer stufenlosen Verschiebbarkeit des Katheterrohrs sind innerhalb eines begrenzten Vorschubabschnitts relativ zum Kapillarrohr, der vorzugsweise zwischen 10 und 30 mm liegt, beliebige Positionierungen ermöglicht. Der Überstand ist manuell ergreifbar, so dass eine Verschiebung des Katheterrohrs mit einem Finger einer Hand erfolgen kann.

In Ausgestaltung der Erfindung ist vorgesehen, dass ein manuell bedienbares Betätigungselement mit dem Überstand, insbesondere dem Stellglied, wirkverbunden ist, das für eine Verlagerung des Überstands, insbesondere des Stellglieds, entlang des Kapillargehäuses beweglich an dem Kapillargehäuse angeordnet ist. Der Überstand, insbesondere das Stellglied, kann mit seiner Außenkontur oder einer Oberflächengestaltung zusätzlich auch das Betätigungselement bilden, oder das Betätigungselement ist ein separates Funktionsteil, das mit dem Überstand, insbesondere dem Stellglied, wirkverbunden ist, oder ein separater Funktionsabschnitt, der fest mit dem Überstand bzw. dem Stellglied verbunden, insbesondere einstückig mit diesem ausgebildet ist.

In weiterer Ausgestaltung der Erfindung ist das Betätigungselement mit dem Stellglied fest verbunden. Das Betätigungselement kann einstückig an dem Stellglied angeformt sein oder in anderer Art und Weise, insbesondere durch Verrastung, an dem Stellglied befestigt sein. Vorteilhaft kann das Betätigungselement durch eine für ein manuelles Ergreifen oder Berühren von wenigstens einem Finger einer Hand eines Anwenders angepasste Kontur oder Oberfläche des Stellgliedes gebildet sein, so dass das Stellglied und das Betätigungselement material- und bauteileinheitlich ausgeführt sind. Die Oberfläche kann eine andere Haptik aufweisen als weitere Flächen des Stellgliedes, um so die Funktion des Betätigungselementes zu erreichen. Alternativ kann das Betätigungselement ein eigenständiges Funktionsteil sein, das mit dem Stellglied einstückig oder über zusätzliche Befestigungsglieder fest verbunden ist.

In weiterer Ausgestaltung der Erfindung wirkt das Betätigungselement mit dem Stellglied indirekt bewegungsübertragend zusammen. Bei dieser Ausgestaltung ist keine feste Verbindung zwischen dem Betätigungselement und dem Stellglied vorgesehen. Vielmehr ist das Betätigungselement an dem Kapillargehäuse beweglich gelagert und mittels eines Antriebsübertragungsglieds wie insbesondere eines Getriebes mit dem Stellglied gekoppelt.

In weiterer Ausgestaltung der Erfindung ist das Betätigungselement verschiebbar an dem Kapillargehäuse gelagert. Hierzu ist an dem Kapillargehäuse eine Linearführungsspur, insbesondere wenigstens ein Führungssteg oder wenigstens eine Führungsnut, vorgesehen, und das Betätigungselement weist wenigstens eine komplementäre Führungsprofilierung auf, die mit der Linearführungsspur zusammenwirkt, um die Verschiebbarkeit des Betätigungselements relativ zum Kapillargehäuse zu erzielen. Die so gebildete Linearführung ist begrenzt, vorzugsweise auf einen Verstellweg zwischen 10 und 30 mm.

In weiterer Ausgestaltung der Erfindung ist das Betätigungselement kraft- oder formschlüssig in wenigstens zwei unterschiedlichen Funktionsstellungen an dem Kapillargehäuse manuell lösbar gehalten. Diese Ausgestaltung ermöglicht eine stufenweise Verschiebbarkeit des Betätigungselements und damit auch des Stellglieds und des Katheterrohrs relativ zum Kapillarrohr. Vorzugsweise weisen das Betätigungselement und das Kapillargehäuse zueinander komplementäre Rastprofilierungen auf, die form- oder kraftschlüssig zusammenwirken, um eine entsprechende Vorschubstellung für das Katheterrohr zu definieren. Die kraft- oder formschlüssigen Funktionsstellungen sind manuell durch eine Hand des Anwenders überdrückbar, so dass der Anwender nach einem Lösen der Klemmeinrichtung das Betätigungselement mit derselben Hand in einfacher Weise in die jeweils nächste Funktionsstellung verlagern kann.

In weiterer Ausgestaltung der Erfindung sind an dem Betätigungselement und an dem Kapillargehäuse zueinander komplementäre Rastprofilierungen vorgesehen, um die wenigstens zwei unterschiedlichen Funktionsstellungen manuell lösbar zu sichern. Die Rastprofilierungen können durch Rastausnehmungen am Kapillargehäuse und durch komplementäre Gestaltung von Rastnocken oder -nasen an einer Außenkontur des Betätigungselements und/oder des Stellglieds gebildet sein, so dass das Betätigungselement und/oder das Stellglied in die jeweils eine Rastausnehmung am Kapillargehäuse formschlüssig einrasten kann.

In weiterer Ausgestaltung der Erfindung ist an dem Kapillargehäuse eine Linearführung zur Verlagerung des Betätigungselements vorgesehen, und das Betätigungselement ist als längs der Linearführung verlagerbarer Betätigungsschieber ausgeführt. Der Betätigungsschieber kann bauteileinheitlich zum Stellglied ausgeführt sein. Die Ausgestaltung realisiert eine stufenlose Verschiebbarkeit des Katheterrohrs relativ zum Kapillargehäuse und relativ zum Kapillarrohr in dem begrenzten Vorschubbereich.

In weiterer Ausgestaltung der Erfindung ist das Betätigungselement rotierbar an dem Kapillargehäuse gelagert, um das Stellglied und damit das Katheterrohr längs des Kapillargehäuses und in dem Kapillarrohr verlagern zu können. Dadurch wird eine indirekte Verschiebung des Katheterrohrs durch eine manuelle Drehbewegung des Betätigungselements erzielt.

In weiterer Ausgestaltung der Erfindung ist das Betätigungselement koaxial zu einer Längsachse des Kapillarrohrs an dem Kapillargehäuse drehbeweglich gelagert und mittels eines Schneckentriebs mit dem Stellglied gekoppelt. Dies ist eine platzsparende und einfach bedienbare Ausgestaltung, da ein gewünschter Vorschub des Katheterrohrs durch eine Drehbewegung des Betätigungselements erzielt wird, die koaxial zu einer Längsachse des Katheterrohrs erfolgt.

In weiterer Ausgestaltung der Erfindung weist die Klemmeinrichtung ein auf das Katheterrohr kraftschlüssig einwirkendes Klemmglied sowie ein am Kapillargehäuse beweglich gelagertes Bedienglied auf, das in einer Klemmstellung das Klemmglied elastisch deformiert, um eine kraftschlüssige Klemmung des Katheterrohrs zu erzielen, und das in einer Freigabestellung das Klemmglied freigibt, so dass das Klemmglied sich in seine unbelastete Ausgangslage zurückstellt, in der das Katheterrohr relativ zu dem Klemmglied beweglich ist. Das Klemmglied kann das Katheterrohr koaxial umschließen. Alternativ kann das Klemmglied von einer Seite her radial auf das Katheterrohr einwirken. Bei allen Varianten wirkt das Bedienglied so auf das Klemmglied ein, dass das Klemmglied relativ zu einer Längsachse des Katheterrohrs eine radiale elastische Klemmung des Katheterrohrs erzielt.

In weiterer Ausgestaltung der Erfindung ist das Bedienglied schwenk- oder drehbeweglich an dem Kapillargehäuse gelagert. Das Bedienglied kann als Hebel, als Bajonettdrehglied, als schwenkbarer Hülsenabschnitt oder in ähnlicher Form ausgestaltet sein.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt schematisch in perspektivischer Darstellung eine erste Ausführungsform einer erfindungsgemäßen Katheteranordnung,
- Fig. 2a und 2b: in zwei unterschiedlichen Funktionsstellungen eine weitere Ausführungsform einer erfindungsgemäßen Katheteranordnung,
- Fig. 3a und 3b: in schematischer perspektivischer Darstellung zwei unterschiedliche Funktionsstellungen einer weiteren Ausführungsform einer erfindungsgemäßen Katheteranordnung,
- Fig. 4: in einer schematischen Längsschnittdarstellung eine weitere Ausführungsform einer erfindungsgemäßen Katheteranordnung,
- Fig. 5: in perspektivischer schematischer Darstellung eine weitere Ausführungsform einer erfindungsgemäßen Katheteranordnung,
- Fig. 6 und 7: in perspektivischer Schemadarstellung zwei unterschiedliche Stellglieder, die auf einem Katheterrohr jeweils einer Ausführungsform einer erfindungsgemäßen Katheteranordnung befestigt sind,
- Fig. 8: in schematischer, perspektivischer Darstellung eine weitere Ausführungsform einer erfindungsgemäßen Katheteranordnung,
- Fig. 9: in einer schematischen Längsschnittdarstellung einen Teilbereich einer weiteren Ausführungsform einer erfindungsgemäßen Katheteranordnung,
- Fig. 10: in perspektivischer, schematischer, teilweise aufgeschnittener Darstellung einen Teilbereich einer weiteren Ausführungsform einer erfindungsgemäßen Katheteranordnung und
- Fig. 11 bis 13: schematisch weitere Ausführungsformen einer erfindungsgemäßen Katheteranordnung.

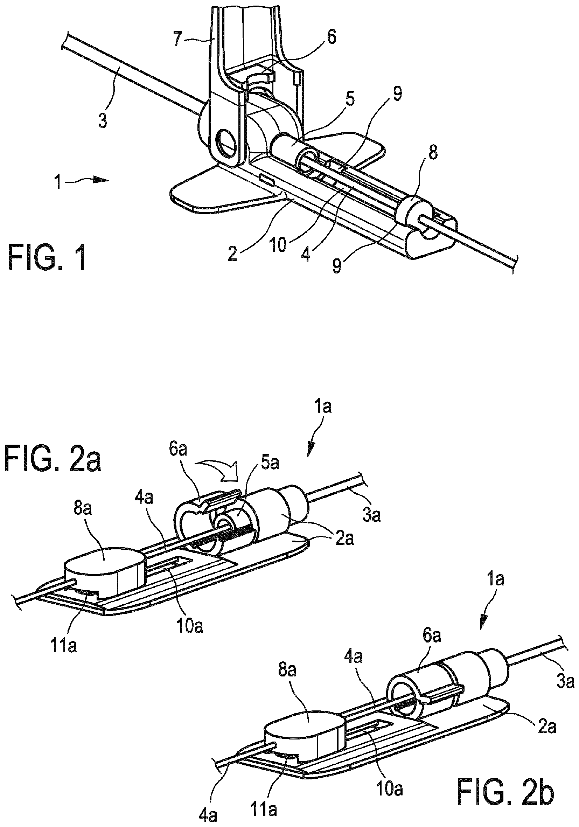

Eine Katheteranordnung 1 nach Fig. 1 ist zur Regionalanästhesie im Bereich der Medizintechnik vorgesehen. Die Katheteranordnung 1 weist ein Kapillargehäuse 2 auf, das aus Kunststoff hergestellt ist. An einem distalen Ende des Kapillargehäuses 2, das auch als Kapillaransatz bezeichnet wird, ist ein Kapillarrohr 3 befestigt, das distal in nicht näher dargestellter Weise mit einer Spitze versehen ist, die in anwendungsbereitem Zustand subkutan einem Patienten zugeführt ist. Um für eine Regionalanästhesie relativ nahe an einzelne, zu blockierende Nerven gelangen zu können, weist die Katheteranordnung 1 zusätzlich ein Katheterrohr 4 auf, das in distaler Richtung proximal in das Kapillarrohr 3 eingeschoben wird und mit einer distalen Spitze sowie einem daran anschließenden Vorschubabschnitt distal aus dem Kapillarrohr 3 herausragt. Eine Länge des über die distale Spitze des Kapillarrohrs 3 hinausragenden Vorschubabschnitts des Katheterrohrs 4 kann in nachfolgend näher beschriebener Weise eingestellt werden. Das Katheterrohr 4 erstreckt sich in Längsrichtung durch das Kapillargehäuse 2 hindurch und kann an seinem nicht dargestellten, proximalen Ende mit einem Verlängerungsschlauch verbunden sein. Das Katheterrohr 4 ist aus Kunststoff als flexibler Schlauch hergestellt.

Das Katheterrohr 4 ist mittels einer nachfolgend näher beschriebenen Verstelleinrichtung bezüglich seiner Vorschublänge, die distal aus dem Kapillarrohr 3 herausragt, einstellbar. Bei der Katheteranordnung 1 gemäß Fig. 1 ist hierzu ein Stellglied 8 in Form einer perlenförmigen Hülse auf einem Außenmantel des Katheterrohrs 4 befestigt. Das Stellglied 8 umschließt das Katheterrohr 4 koaxial und weist einen zumindest weitgehend zylindrischen Außenmantel auf. Das Kapillargehäuse 2 weist zwei halbzylindrische Vertiefungen 9 auf, die in Längsrichtung des Katheterrohrs 4 zueinander beabstandet sind. Die Ausnehmungen 9 sind auf den Außenmantel des Stellglieds 8 abgestimmt, um ein lösbares Einrasten des Stellglieds 8 in einer dieser Ausnehmungen 9 zu ermöglichen. Die beiden zueinander beabstandeten Ausnehmungen 9 sind über eine rinnenartige Linearführung 10 miteinander verbunden, die - wie die Ausnehmungen 9 - einstückiger Bestandteil des Kapillargehäuses 2 ist. In der in Fig. 1 dargestellten Position ist das Katheterrohr 4 in Längsrichtung relativ zum Kapillargehäuse 2 positioniert, indem das Stellglied 9 in die proximale Ausnehmung 9 formschlüssig eingetaucht ist. Wenn ein Vorschub des Katheterrohrs 4 relativ zum Kapillarrohr 3 verändert werden soll, kann das Stellglied 8 in einfacher Weise durch einen Anwender mit zwei Fingern einer Hand angehoben und längs der rinnenförmigen Linearführung 10 distal entlang des Kapillargehäuses 2 verschoben werden, bis das Stellglied 8 in die zweite Ausnehmung 9 des Kapillargehäuses 2 eintaucht. Die Katheteranordnung 1 bildet demzufolge eine zweistufige Vorschubmöglichkeit für das Katheterrohr 4.

Um das Katheterrohr 4 relativ zum Kapillargehäuse 2 zusätzlich fixieren zu können, ist eine Klemmeinrichtung vorgesehen, die ein elastisch deformierbares Klemmglied 5 aufweist, das das Katheterrohr 4 koaxial hülsenförmig umschließt und in das Kapillargehäuse 2 distal benachbart zu der distalen Ausnehmung 9 eingebettet ist. In einer in Fig. 1 dargestellten Freigabestellung der Klemmeinrichtung umschließt das Klemmglied 5 das Katheterrohr 4 in radialem Abstand. Dies stellt die unbelastete Ausgangslage des Klemmglieds 5 dar. Um eine Klemmung des Katheterrohrs 4 durch das Klemmglied 5 zu erzielen, ist ein in Fig. 1 als schwenkbeweglich am Kapillargehäuse 2 gelagerter Bedienhebel ausgeführtes Bedienglied 7 vorgesehen, das mit einem Kraftübertragungsabschnitt 6 versehen ist, der beim Zuschwenken des Bedienglieds 7 das Klemmglied 5 von oben her - auf die Darstellung in Fig. 1 bezogen - radial elastisch deformiert. Dadurch wird das Klemmglied 5 radial gegen den Außenmantel des Katheterrohrs 4 gepresst, wodurch die gewünschte Klemmwirkung erzielt wird, die eine axiale Fixierung des Katheterrohrs 4 relativ zum Kapillargehäuse 2 bewirkt. Eine Klemmung des Katheterrohrs 4 relativ zum Kapillargehäuse 2 mittels des Klemmglieds 5 kann auch in einer Zwischenstellung des perlenförmigen Stellglieds 8 erfolgen, in der das Stellglied 8 in der rinnenförmigen Linearführung 10 zwischen den beiden Ausnehmungen 9 des Kapillargehäuses 2 aufliegt.

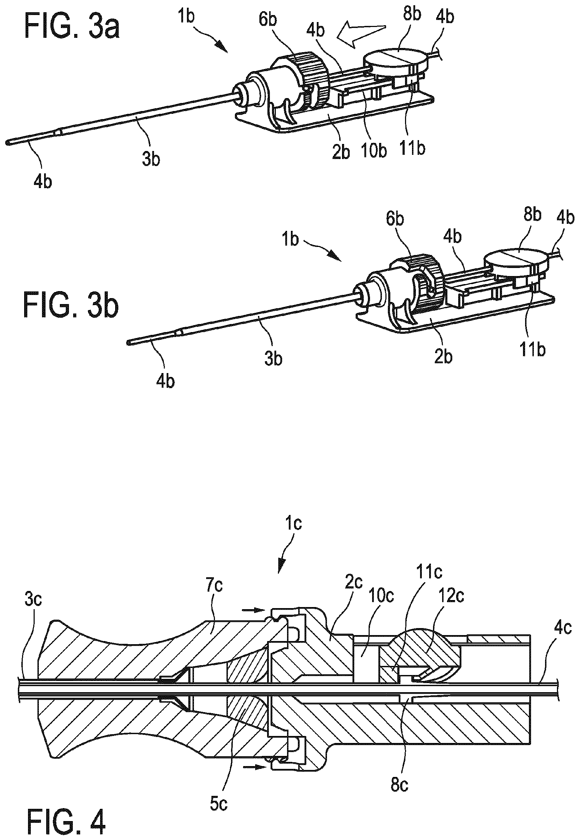

Die Katheteranordnung 1a nach den Fig. 2a und 2b entspricht vom funktionalen Aufbau her der Katheteranordnung 1 nach Fig. 1. Funktionsgleiche Bauteile und Abschnitte sind daher mit gleichen Bezugszeichen unter Hinzufügung des Buchstabens a versehen. Auch die Katheteranordnung 1a dient in analoger Weise zur Regionalanästhesie. Zur Vermeidung von Wiederholungen wird ergänzend auf die Ausführungen zur Katheteranordnung 1 nach Fig. 1 verwiesen. Gleiches gilt auch für die nachfolgend anhand der Fig. 3a bis 10 beschriebenen Ausführungsformen erfindungsgemäßer Katheteranordnungen. Auch dort sind jeweils funktionsgleiche Bauteile oder Abschnitte mit gleichen Bezugszeichen unter Hinzufügung eines jeweils nachfolgenden Kleinbuchstabens b bis i versehen. Die Anwendung dieser Katheteranordnungen 1b bis 1g entspricht der Anwendung der Katheteranordnungen 1 und 1a, so dass zur Vermeidung von Wiederholungen auch bezüglich dieser nachfolgend näher beschriebenen Ausführungsformen auf die vorstehenden Ausführungen zur Katheteranordnung 1 ergänzend verwiesen wird.

Die Katheteranordnung 1a weist ein Kapillargehäuse 2a auf, das distal fest mit einem Kapillarrohr 3a verbunden ist. Ein Katheterrohr 4a ragt koaxial in distaler Richtung durch das Kapillarrohr 3a hindurch. Das Katheterrohr 4a ist von einem Stellglied 8a umschlossen, das mittels eines Führungselements 11a in einer schlitzartigen Linearführung 10a längs des Kapillargehäuses 2a begrenzt verschiebbar gelagert ist. Das Stellglied 8a ist blockartig gestaltet und fest mit dem Katheterrohr 4a verbunden. Das Stellglied 8a bildet zusätzlich ein Betätigungselement im Sinne der Erfindung mit seiner Außenkontur, die manuell von einer Hand eines Anwenders ergreifbar ist. Die Linearführung 10a ist in einem Trägerplattenabschnitt des Kapillargehäuses 2a vorgesehen und begrenzt eine Linearverschiebbarkeit des Stellglieds 8a auf einen Vorschubweg, der vorzugsweise zwischen 15 mm und 30 mm beträgt. Die Linearführung 10a erstreckt sich in Längsrichtung des Kapillargehäuses 2a und damit parallel zu dem Katheterrohr 4a. Das Führungselement 11a ist nach Art eines Kulissenstegs in der Linearführung 10a das Kapillargehäuses 2a gehalten, wobei die Führung derart gestaltet ist, dass das Stellglied 8a selbsthemmend relativ zur Linearführung 10a positioniert bleibt, sobald eine manuelle Schubkraft durch die Hand eines Anwenders entfällt. Das Stellglied 8a ist demzufolge begrenzt kraftschlüssig in der Linearführung 10a verschiebbar. Durch eine Verschiebung des Stellglieds 8a ist ein Vorschub eines distalen Abschnitts des Katheterrohrs 4a, der über eine distale Spitze des Kapillarrohrs 3a hinausragt, in den Grenzen der Linearführung 10a stufenlos verstellbar. Das Führungselement 11a kann in der Linearführung 10a des Kapillargehäuses 2a auch mit Spiel linearbeweglich verschiebbar sein. Denn eine zusätzliche Sicherung des Katheterrohrs 4a relativ zum Kapillargehäuse 2a und damit auch relativ zum Kapillarrohr 3a erfolgt durch eine Klemmeinrichtung, die ein elastisch deformierbares, hülsenförmiges Klemmglied 5a aufweist, das das Katheterrohr 4a koaxial und radial beabstandet umschließt. Als Bedienglied 6a zur elastischen Deformation des Klemmglieds 5a ist ein Bügel vorgesehen, der um eine zur Längsachse des Katheterrohrs 4a parallele Schwenkachse an dem Kapillargehäuse 2a schwenkbeweglich gelagert ist zwischen einer in Fig. 2a dargestellten Freigabeposition und einer in Fig. 2b gezeigten Klemmposition. In der Klemmposition presst eine Innenseite des kreisbogenförmigen Bügels 6a das hülsenförmige, elastisch deformierbare Klemmglied 5a zusammen, wodurch eine radiale Klemmung des Katheterrohrs 4a erfolgt. Das Bedienglied 6a ist mittels eines Filmscharniers einstückig an dem Kapillargehäuse 2a angeformt. In der Klemmstellung ist für das Bedienglied 6a ein Rastmechanismus vorgesehen, der ebenfalls einstückig am Kapillargehäuse 2a angeformt ist und das Bedienglied 6a in der Klemmstellung am Kapillargehäuse 2a manuell lösbar sichert. Das Stellglied 8a ist ausreichend groß gestaltet, um eine Verschiebung mithilfe wenigstens eines Fingers einer Hand eines Anwenders längs des Kapillargehäuses 2a zu ermöglichen.

Die Katheteranordnung 1b nach den Fig. 3a und 3b entspricht vom funktionalen Aufbau her der Katheteranordnung 1a nach den Fig. 2a und 2b. Wesentlicher Unterschied ist es, dass für die Verstelleinrichtung zur Linearführung des Stellglieds 8b am Kapillargehäuse 2b als Funktionsspur eine geradlinige Stegführung 10b vorgesehen ist, auf der das Stellglied 8b mittels eines am Stellglied 8b einstückig angeformten Führungselements 11b in Form eines Reiters begrenzt längsverschiebbar geführt ist. Das Katheterrohr 4b wird mittels des Stellglieds 8b längs des Kapillarrohrs 3b des Kapillargehäuses 2b linearverschiebbar verlagert, wobei ein distal aus dem Kapillarrohr 3b herausragender Vorschubabschnitt in seiner Länge veränderbar ist abhängig von der Schiebestellung des Stellglieds 8b relativ zu dem Kapillargehäuse 2b.

Auch die Katheteranordnung 1b nach den Fig. 3a und 3b weist als Teil einer Klemmeinrichtung ein hülsenförmiges, elastisch deformierbares Klemmglied auf, das im Bereich des Kapillargehäuses 2b positioniert ist und das Katheterrohr 4b koaxial umschließt. In einer Freigabestellung umschließt das Klemmglied das Katheterrohr 4b in radialem Abstand. Die Klemmeinrichtung weist ein koaxial zu einer Längsachse des Kapillarrohrs 3b drehbeweglich am Kapillargehäuse 2b gelagertes Bedienglied 6b auf, das mittels einer Bajonettfunktion eine axiale Relativbeweglichkeit auf das Klemmglied bewirkt. Dabei wird das Klemmglied mittels eines nicht sichtbaren Konus in radialer Richtung elastisch deformiert und Klemmung des Katheterrohrs 4b. Sobald das Bedienglied 6b wieder in entgegengesetzter Drehrichtung zurückgedreht wird, erfolgt wieder das Lösen des Klemmglieds von dem Außenmantel des Katheterrohrs 4b.

Die Katheteranordnung 1c nach Fig. 4 weist ebenfalls eine Verstelleinrichtung zur Einstellung des Vorschubs des Katheterrohrs 4c relativ zum Kapillarrohr 3c auf, das relativ zu einem Kapillargehäuse 2c fest positioniert ist. Allerdings ist bei der Ausführungsform nach Fig. 4 das Kapillarrohr 3c an einem als Bedienglied einer Klemmeinrichtung für das Katheterrohr 4c fungierenden Gehäusefortsatz 7c des Kapillargehäuses 2c befestigt. Der Gehäusefortsatz 7c ist relativ zum Kapillargehäuse 2c in Richtung der in Fig. 4 erkennbaren Pfeile begrenzt längsverschiebbar ausgeführt. Innenseitig nimmt der Gehäusefortsatz 7c das elastisch deformierbare Klemmglied 5c auf, das bei einer zum Katheterrohr 4c koaxialen Relativverschiebung zwischen Gehäusefortsatz 7c und Kapillargehäuse 2c aufgrund einer konischen Aufnahme im Gehäusefortsatz 7c zwangsläufig radial nach innen zusammengepresst wird, wodurch die gewünschte Klemmwirkung auf den Außenmantel des Katheterrohrs 4c erzeugt wird. Bei gelöstem Klemmglied 5c ist das Katheterrohr 4c mittels einer Verstelleinrichtung in seinem Vorschub relativ zum Kapillarrohr 3c veränderbar. Hierzu ist auf dem Außenmantel des Katheterrohrs 4c ein ringförmiges Stellglied 8c befestigt, das in einer Linearführung 10c des Kapillargehäuses 2c längsverschiebbar gelagert ist. An dem Stellglied 8c greift ein schieberförmiges Betätigungselement 12c an, das formschlüssig mit dem Stellglied 8c verbunden ist und ebenfalls in der Linearführung 10c des Kapillargehäuses 2c parallel zur Längsachse des Katheterrohrs 4c begrenzt verschiebbar geführt ist. Das Betätigungselement 12c weist ein stegförmiges Führungselement 11c auf, das zum einen eine distale Stirnseite des Stellglieds 8c formschlüssig blockiert und zum anderen in der Linearführung 10c des Kapillargehäuses 2c längsverschiebbar geführt ist. Zudem sind zwei elastisch nachgiebige, am Betätigungselement 12c einstückig angeformte, nicht näher bezeichnete Finger vorgesehen, die das gegenüberliegende Stirnende des Stellglieds 8c gegen den Führungssteg 11c stützen. Die beiden Finger sind derart geneigt, dass das Katheterrohr 4c von einem proximalen Ende des Kapillargehäuses 2c her in das Kapillargehäuse 2c und anschließend durch den Gehäusefortsatz 7c in das Kapillarrohr 3c in distaler Richtung einschiebbar ist. Die beiden Finger weichen aus und rasten hinter dem Stellglied 8c wieder ein, so dass das Katheterrohr 4c relativ zum Betätigungselement 12c gesichert ist.

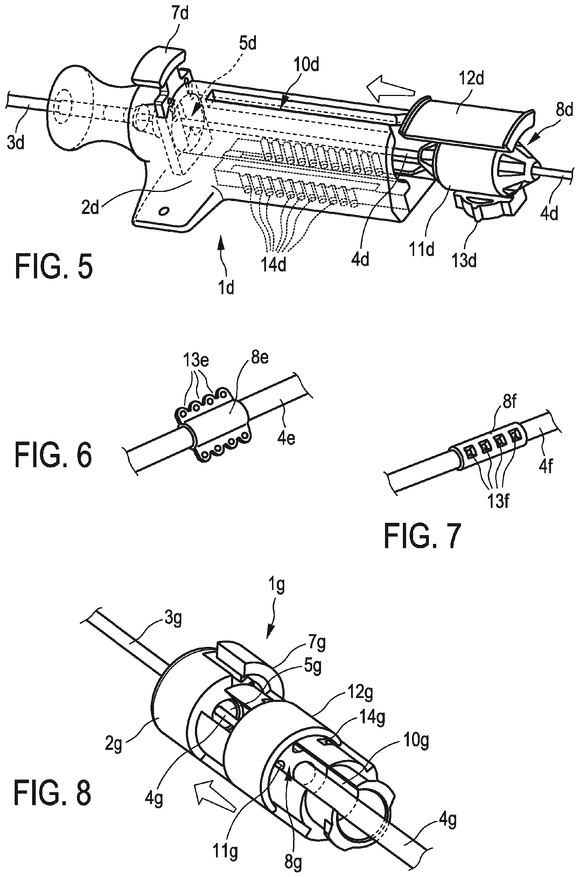

Bei der Katheteranordnung 1d gemäß Fig. 5 ist in einem Kapillargehäuse 2d eine Linearführung 10d vorgesehen, die rohrartig koaxial zu einer Längsachse des distal am Kapillargehäuse 2d befestigten Kapillarrohrs 3d verläuft. Das Katheterrohr 4d ist von einem Stellglied 8d umschlossen, das zusätzlich ein teilzylindrisches Führungselement 11d ausbildet, das in der Linearführung 10d des Kapillargehäuses 2d längsverschiebbar geführt ist. An dem Stellglied 8d ist zudem als Betätigungselement 12d ein Schieber einstückig angeformt, der in einem Längsschlitz der Linearführung 10d des Kapillargehäuses 2d geführt ist und zu einer Oberseite über das Kapillargehäuse 2d hinausragt. Auf einer gegenüberliegenden Unterseite ist das Stellglied 8d beidseitig mit jeweils einem einstückig angeformten, elastisch nachgiebigen Raststeg 13d versehen, dem innenseitig des Kapillargehäuses 2d komplementäre Rastaussparungen 14d zugeordnet sind. Dadurch ist eine der Anzahl der Rastaussparungen 14d entsprechende Anzahl von Raststufen für einen Vorschub des Katheterrohrs 4d relativ zum Kapillarrohr 3d gewährleistet. Die in die jeweilige Rastaussparung 14d eingreifenden Raststege 13d auf gegenüberliegenden Seiten des Stellglieds 8d sichern die jeweils eingestellte Vorschubposition. Zudem wird das Katheterrohr 4d bei Bedarf axial fixiert durch die Klemmeinrichtung, die ein Bedienglied 7d und ein radial elastisch deformierbares Klemmglied 5d aufweist. Das Bedienglied 7d ist nach Art eines Schubstifts orthogonal zur Längsachse des Katheterrohrs 4d in dem Kapillargehäuse 2d begrenzt hubbeweglich geführt. Das Bedienglied 7d wirkt auf das Klemmglied 5d radial ein, wodurch dieses radial zusammengepresst und gegen den Außenmantel des Katheterrohrs 4d gedrückt wird. Dem Bedienglied 7d ist in nicht näher dargestellter Weise eine Raststellung für die Klemmstellung im Kapillargehäuse 2d zugeordnet, in der das Klemmglied 5d das Katheterrohr 4d festklemmt.

Bei den Ausführungsformen gemäß den Fig. 6 und 7 sind Stellglieder 8e und 8f auf dem jeweiligen Katheterrohr 4e und 4f befestigt, die jeweils mit mehreren in Längsrichtung des Katheterrohrs 4e, 4f erstreckten Rastprofilierungen 13e und 13f versehen sind. In nicht näher dargestellter Weise weist das Kapillargehäuse hierzu komplementäre, bewegliche Rastelemente auf, wodurch das Stellglied 8e, 8f in unterschiedlichen Raststellungen in Vorschubrichtung des Katheterrohrs 4e, 4f manuell lösbar relativ zum Kapillargehäuse fixierbar ist.

Die Katheteranordnung 1g gemäß Fig. 8 entspricht funktional weitgehend der Katheteranordnung 1d nach Fig. 5. Auch hier ist ein ringförmiges Stellglied 8g auf einem Außenmantel des Katheterrohrs 4g befestigt, das in dem Kapillargehäuse 2g und dem daran befestigten Kapillarrohr 3g längsverschiebbar angeordnet ist. Bei der Katheteranordnung 1g ist das Stellglied 8g am Übergang des Katheterrohrs 4g zu einem Verlängerungsschlauch fixiert, der proximal zu einem distalen Rohrabschnitt des Katheterrohrs 4g befestigt ist. Das Stellglied 8g ist mit einer ringförmigen Führungsfläche in einer zylindrischen Linearführung 10g des Kapillargehäuses 2g längsverschiebbar geführt. Als Betätigungselement ist ein Schieber 12g vorgesehen, der mittels eines Führungsstegs 11g einstückig mit dem Stellglied 8g verbunden ist. Das Führungsglied 11g ragt durch einen Längsschlitz der Linearführung 10g des Kapillargehäuses 2g nach oben hindurch. Der Schieber 12g ist außenseitig auf einem zylindrischen Gehäuseabschnitt des Kapillargehäuses 2g längsverschiebbar geführt. Am zylindrischen Gehäuseabschnitt des Kapillargehäuses 2g sind mehrere in Längsrichtung in gleichmäßigen Abständen hintereinander angeordnete Rastausnehmungen 14g vorgesehen. Der Schieber 12g weist wenigstens eine komplementäre, nicht näher dargestellte Rastnase auf, die in die jeweilige Rastausnehmung 14g des Kapillargehäuses 2g abhängig von einer Vorschubstellung des Katheterrohrs 4g relativ zum Kapillarrohr 3g manuell lösbar einrastet. Zudem ist im Kapillargehäuse 2g distal ein hülsenförmiges Klemmglied 5g vorgesehen, das analog der zuvor beschriebenen Klemmglieder elastisch deformierbar ausgeführt ist und das Katheterrohr 4g koaxial umschließt. Ein Bedienglied 7g ist ähnlich der Ausführungsform nach Fig. 5 radial hubbeweglich an dem Kapillargehäuse 2g gelagert, um für eine Klemmfunktion radial nach innen gedrückt zu werden unter radialer Deformation des Klemmglieds 5g. Dadurch wird das Katheterrohr 4g radial geklemmt und relativ zum Kapillargehäuse 2g gesichert.

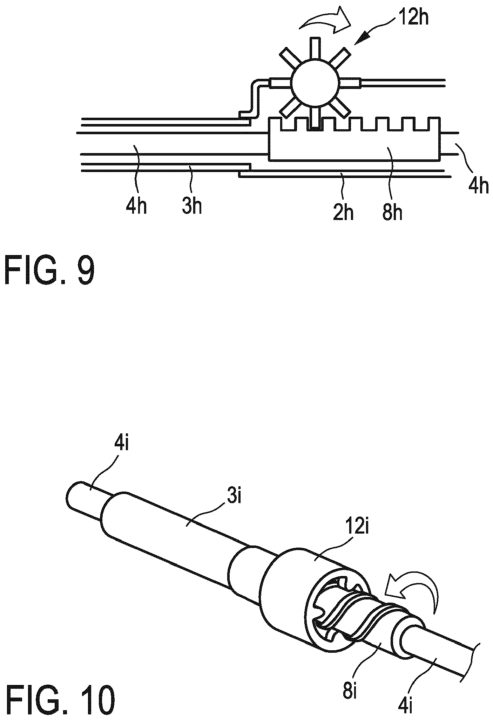

Bei der Ausführungsform nach Fig. 9 ist ein Betätigungselement 12h einer Verstelleinrichtung zur Veränderung des Vorschubs des Katheterrohrs 4h um eine zur Vorschubrichtung des Katheterrohrs 4h orthogonale Drehachse drehbeweglich am Kapillargehäuse 2h gelagert. Das Stellglied 8h weist auf einer dem Betätigungselement 12h zugewandten Längsseite eine Zahnstangenfunktion auf. Das Betätigungselement 12h ist mit einer komplementären Stirnverzahnung versehen, die mit der Zahnstangenfunktion des Stellglieds 8h kämmt, um so die gewünschte Vorschubfunktion für das Katheterrohr 4h im Kapillarrohr 3h zu erzielen.

Bei der Ausführungsform nach Fig. 10 wird eine Vorschubbewegung des Katheterrohrs 4i relativ zu einem Kapillarrohr 3i erzielt durch ein Betätigungselement 12i, das koaxial zu dem Kapillarrohr 3i drehbeweglich an einem nicht näher dargestellten Kapillargehäuse gelagert ist. Das Stellglied 8i weist an seinem Außenumfang eine Gewindeschnecke auf, die mit einem Innengewinde des Betätigungselements 12i zusammenwirkt. Eine relative Drehbewegung zwischen dem Betätigungselement 12i und dem Stellglied 8i führt so zwangsläufig zu einer stufenlosen Vorschubbewegung in distaler Richtung oder in proximaler Richtung, je nach Relativdrehrichtung zwischen dem Betätigungselement 12i und dem Stellglied 8i.

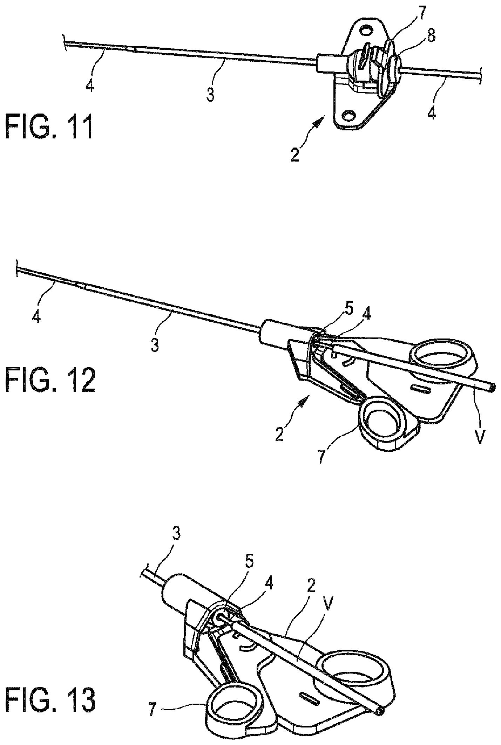

Bei einer Katheteranordnung gemäß Fig. 11 ist an einem Kapillargehäuse 2 distal ein Kapillarrohr 3 befestigt, in dem ein Katheterrohr 4 längs verschiebbar geführt ist. Distal des Kapillargehäuses ist auf einem Außenmantel des Katheterrohrs 4 ein perlenförmiges Stellglied 8 befestigt, dass einen relativ zu einer Längsachse des Katheterrohrs 4 radialen Überstand bildet. In nicht näher dargestellter Weise ist in dem Kapillargehäuse 2 ein elastisch deformierbares Klemmglied vorgesehen, dass mittels eines drehbaren Betätigungsflügels 7 verdrillbar ist, um eine elastische Klemmung des Katheterrohrs 4 zu erzielen. Ein Anwender kann somit mit einer Hand bei gelöster Klemmeinrichtung an dem Stellglied 8 angreifen und das Katheterrohr 4 relativ zum Kapillarrohr 3 längs verlagern. Der entsprechend eingestellte Vorschub kann durch ein anschließendes Verdrehen des Betätigungsflügels 7 mit derselben Hand fixiert werden, da durch das Verdrehen des Betätigungsflügels 7 das Klemmglied radial von außen her eine Klemmkraft auf den Außenmantel des Katheterrohrs 4 aufbringt.

Bei der Katheteranordnung nach den Fig. 12 und 13 wird auf ein proximales Ende eines Katheterrohrs 4 ein Verlängerungsschlauch V mit größerem Durchmesser aufgeschoben und auf dem proximalen Stirnende des Katheterrohrs 4 kraftschlüssig fixiert. Da der Verlängerungsschlauch V einen größeren Durchmesser aufweist als das Katheterrohr 4 wird zwangsläufig durch das aufgeschobene distale Stirnende des Verlängerungsschlauches V ein radialer Überstand am Katheterrohr 4 geschaffen, der bei einer Verschiebung des Katheterrohrs zwangsläufig selbst relativ zum Kapillargehäuse 2 begrenzt verschiebbar geführt ist. Das Kapillargehäuse 2 weist ein distal an dem Kapillargehäuse 2 angebrachtes Kapillarrohr 3 auf. Koaxial zum Katheterrohr 4 ist am Kapillargehäuse 2 ein radial elastisch deformierbares, hülsenförmiges Klemmglied 5 vorgesehen, dass distal einen axialen Anschlag für den radialen Überstand des Verlängerungsschlauches V bildet. Das Klemmglied 5 als Teil einer Klemmeinrichtung wird durch einen als Betätigungselement 7 dienenden Scherenflügel elastisch deformiert, der relativ zu dem Kapillargehäuse 2 schwenkbeweglich ist. Das Kapillargehäuse 2 weist als Gegenflügel zu der Scherenfunktion des Scherenflügels einen einstückig angeformten Eingriff auf, in den ein Finger einer Hand eines Anwenders eingreifen kann. Mit zwei Fingern einer Hand ist somit der Scherenflügel relativ zu dem Gegenflügel schwenkbeweglich, um das Klemmglied elastisch zu deformieren und so das Katheterrohr 4 relativ zum Kapillargehäuse 2 axial zu fixieren. Mit derselben Hand kann der Verlängerungsschlauch V vor dem Klemmen des Klemmgliedes 5 ergriffen und ein Vorschub des Katheterrohrs 4 relativ zum Kapillarrohr 3 eingestellt werden. Entsprechende Raststellen am Betätigungsflügel 7 einerseits und am Kapillargehäuse 2 andererseits können bei einem Zusammendrücken der Scherenteile die elastische Deformation des Klemmgliedes 5 und damit die Klemmstellung des Scherenflügels sichern, sodass eine axiale Fixierung des Katheterrohrs 4 aufrechterhalten bleibt.

## Patentansprüche

1. Katheteranordnung (1 bis 1g) für eine Regionalanästhesie, mit einem Kapillargehäuse (2 bis 2h), an dem distal ein Kapillarrohr (3 bis 3i) vorgesehen ist, sowie mit einem Katheterrohr (4 bis 4i), das proximal in das Kapillarrohr (3 bis 3i) eingeführt ist und abschnittsweise distal aus dem Kapillarrohr (3 bis 3i) herausragt, wobei das Katheterrohr (4 bis 4i) in dem Kapillarrohr (3 bis 3i) begrenzt linearbeweglich verschiebbar angeordnet ist, sowie mit einer manuell betätigbaren Klemmeinrichtung zur lösbaren Fixierung des Katheterrohrs (4 bis 4i) relativ zu dem Kapillarrohr (3 bis 3i) und dem Kapillargehäuse (3 bis 3h), **dadurch gekennzeichnet, dass** zusätzlich eine Verstelleinrichtung am Kapillargehäuse (2 bis 2h) vorgesehen ist, die eine stufenweise oder stufenlose manuelle Verschiebung des Katheterrohrs (4 bis 4i) im Kapillarrohr (3 bis 3i) gewährleistet, wobei an dem Katheterrohr (4 bis 4i) wenigstens ein manuell ergreifbarer radialer Überstand, insbesondere ein Stellglied (8 bis 8i), vorgesehen ist, der entlang des Kapillargehäuses (2 bis 2h) begrenzt verstellbar geführt ist.

2. Katheteranordnung (1 bis 1g) nach Anspruch 1, **dadurch gekennzeichnet, dass** ein manuell bedienbares Betätigungselement (12c bis 12i) mit dem Überstand, insbesondere dem Stellglied (8 bis 8i), wirkverbunden ist, das für eine Verlagerung des Überstandes, insbesondere des Stellglieds (8 bis 8i), entlang des Kapillargehäuses (2 bis 2h) an dem Kapillargehäuse (2 bis 2h) beweglich angeordnet ist.

3. Katheteranordnung (1, 1a, 1b, 1d, 1g) nach Anspruch 1 oder 2, **dadurch gekennzeichnet, dass** das Betätigungselement (12d, 12g) mit dem Stellglied (8, 8a, 8b, 8d, 8g) fest verbunden ist.

4. Katheteranordnung nach Anspruch 2, **dadurch gekennzeichnet, dass** das Betätigungselement (12c, 12h, 12i) mit dem Stellglied (8c) bewegungsübertragend, insbesondere indirekt, zusammenwirkt.

5. Katheteranordnung (1, 1a, 1b, 1c, 1d, 1g) nach einem der vorhergehenden Ansprüche, **dadurch gekennzeichnet, dass** das Betätigungselement (12c, 12d, 12g) verschiebbar an dem Kapillargehäuse (2, 2a, 2b, 2c, 2d, 2g) gelagert ist.

6. Katheteranordnung (1, 1d, 1g) nach einem der vorhergehenden Ansprüche, **dadurch gekennzeichnet, dass** das Betätigungselement kraft- oder formschlüssig in wenigstens zwei unterschiedlichen Funktionsstellungen an dem Kapillargehäuse (2, 2d, 2g) manuell lösbar gehalten ist.

7. Katheteranordnung (1d, 1g) nach Anspruch 6, **dadurch gekennzeichnet, dass** an dem Betätigungselement (12d, 12g) und an dem Kapillargehäuse (2d, 2g) zueinander komplementäre Rastprofilierungen vorgesehen sind, um die wenigstens zwei unterschiedlichen Funktionsstellungen manuell lösbar zu sichern.

8. Katheteranordnung (1a, 1b, 1c, 1d, 1g) nach einem der vorhergehenden Ansprüche, **dadurch gekennzeichnet, dass** an dem Kapillargehäuse (2a, 2b, 2c, 2d, 2g) eine Linearführung (10a, 10b, 10c, 10d, 10g) zur Verlagerung des Betätigungselements (12c, 12d, 12g) vorgesehen ist, und dass das Betätigungselement (12c, 12d, 12g) als längs der Linearführung (10a, 10b, 10c, 10d, 10g) verlagerbarer Betätigungsschieber ausgeführt ist.

9. Katheteranordnung nach einem der vorhergehenden Ansprüche, **dadurch gekennzeichnet, dass** das Betätigungselement (12h, 12i) rotierbar an dem Kapillargehäuse (2h) gelagert ist, um das Stellglied (8h, 8i) und damit das Katheterrohr (4h, 4i) längs des Kapillargehäuses (2h) und in dem Kapillarrohr (3h, 3i) verlagern zu können.

10. Katheteranordnung nach Anspruch 9, **dadurch gekennzeichnet, dass** das Betätigungselement (12i) koaxial zu einer Längsachse des Kapillarrohrs (3i) an dem Kapillargehäuse drehbeweglich gelagert ist und mittels eines Schneckentriebs mit dem Stellglied (8i) gekoppelt ist.

11. Katheteranordnung nach Anspruch 9, **dadurch gekennzeichnet, dass** das Betätigungselement (12h) um eine zu der Längsachse des Kapillarrohrs (3h) orthogonale Drehachse drehbeweglich an dem Kapillargehäuse (2h) gelagert ist, und dass das Betätigungselement (12h) mittels eines Zahnstangentriebs mit dem Stellglied (8h) gekoppelt ist.

12. Katheteranordnung (1 bis 1g) nach einem der vorhergehenden Ansprüche, **dadurch gekennzeichnet, dass** die Klemmeinrichtung ein auf das Katheterrohr (4 bis 4g) kraftschlüssig einwirkendes Klemmglied (5 bis 5g) sowie ein am Kapillargehäuse (2 bis 2g) beweglich gelagertes Bedienglied (6 bis 6g) aufweist, das in einer Klemmstellung das Klemmglied (5 bis 5g) elastisch deformiert, um eine kraftschlüssige Klemmung des Katheterrohrs (4 bis 4g) zu erzielen, und das in einer Freigabestellung das Klemmglied (5 bis 5g) freigibt, so dass das Klemmglied (5 bis 5g) sich in seine unbelastete Ausgangslage zurückstellt, in der das Katheterrohr (4 bis 4g) relativ zu dem Klemmglied (5 bis 5g) beweglich ist.

13. Katheteranordnung nach Anspruch 1, **dadurch gekennzeichnet, dass** das Bedienglied (6, 7, 6a, 6b, 7c, 7d, 7g) linear-, schwenk- oder drehbeweglich an dem Kapillargehäuse (2 bis 2g) gelagert ist.
